# EUROPEAN PATENT APPLICATION

(11) **EP 1 179 352 A1**
(43) Date of publication of application: **13.02.2002**
(21) Application number: 00830567.4
(22) Date of filing: 08.08.2000
(51) Int. Cl.: A61L 26/00, A61L 15/22

(54) **Topical composition for covering a skin lesion**

(71) Applicant: MONTERESEARCH S.r.l., 20016 Pero (IT)
(72) Inventor: Ronchi, Celestino, 20127 Milano (IT); Porziotta, Enrico, 20159 Milano (IT); Farinelli, Claudio, 20016 Pero (MI) (IT); Baffi, Gottardo, 20161 Milano (IT)
(74) Representative: Marchi, Massimo

(57) **Abstract**

A topical film-forming aqueous composition for covering a skin lesion comprising a mixture of a polyvinyl alcohol and of a chitosan and capable of forming a dry film *in situ.*

## Description

The present invention relates to a topical composition for covering a skin lesion.

In particular, the present invention relates to a topical composition capable of covering a skin lesion by forming a dry film *in situ.*

It is known that plasters are commonly employed for covering skin lesions.

A plaster should possess good adhesiveness, breaking strength and elasticity.

Such properties are necessary mainly so that the plaster resists the movements of the zone on which it has been placed.

In general, a conventional plaster is formed from a support of fabric or plastic covered, at the ends, with an adhesive composition and, at the centre, with gauze.

The gauze is centred on the damaged area and the adhesive support holds it in place.

As well as performing a protective action by isolating the damaged part from the environment, this gauze also produces a medicinal effect when it has previously been treated with agents such as disinfectants, antiseptics and healing agents.

However, plasters have many drawbacks.

A first drawback is that the adhesion of the adhesive support varies over time. This is manifested initially in excessive adhesion to the skin so that the plaster is difficult to remove, causing irritation of the skin. In contrast, when the plaster gets old, its adhesiveness decreases to the point where it becomes practically useless.

A second drawback is that a first-aid box would need to contain various shapes and sizes of plasters. Moreover, this increases the likelihood that a large number of them will become unusable because of the decrease in adhesiveness over time.

A third drawback is that self-application of a plaster is often complicated. This is particularly so when the lesion to be covered is at a location that cannot be reached by both hands of the person who has to medicate himself. This occurs, for example, when the lesion is on an arm or on the person's back.

A fourth drawback is that some individuals exhibit intolerance to the adhesives used in plaster manufacture.

A fifth drawback, of an aesthetic nature, which has not yet been solved satisfactorily with conventional plasters, is their visibility.

Now it has been found surprisingly that a conventional plaster can be replaced by a topical film-forming composition capable of uniformly covering a skin lesion by *in situ* formation of a dry, elastic film.

This is even more surprising in view of the fact that the said composition comprises polyvinyl alcohol and chitosan and it is known that these have properties of breaking strength and elasticity that are so different from one another that the results described below were not predictable. Indeed, polyvinyl alcohol has good elastic properties but poor breaking strength, whereas chitosan exhibits opposite properties. Their aqueous association, however, leads to a dry film whose breaking strength and elastic properties are greater than the simple sum of the two components taken individually (see Tables 1 and 2).

It is, therefore, a first object of the present invention is a topical aqueous film-forming composition for covering a skin lesion, characterized in that it comprises a mixture of a polyvinyl alcohol and a chitosan and is capable of forming a dry film *in situ.*

As is known, polyvinyl alcohol can be represented by the formula (C₂H₄O)ₙ in which n preferably has a value of from 500 to 5000. The amount of polyvinyl alcohol in the topical film-forming composition is preferably of from 1 to 30 by weight % and even more preferably of from 5 to 15 by weight %.

Preferably, the said chitosan has a molecular weight of from 300,000 to 2,000,000. Even more preferably, it also has a deacetylation degree of at least 80%. The amount of chitosan in the topical film-forming composition is preferably of from 0.15 to 5 by weight % and even more preferably of from 0.5 to 3 by weight %.

A polyvinyl alcohol/chitosan weight ratio of 6.6 : 1 is particularly preferred.

Advantageously, the said composition further comprises at least one disinfectant. Typical examples of said disinfectants are listed in "*Remington's Pharmaceutical Sciences*", 8th edition, Alfonso R Gennaro et al.; *"Modern Pharmacology",* 4th edition, Charles R. Craig and Robert E. Stitzer and "*Martindale*", 2-3rd, Kathleen Partfitt.

They belong to various chemical classes such as, for example, acids, alcohols, aldehydes, halogens and halogenated compounds, heavy metals compounds, phenols, and quaternary ammonium compounds.

Preferred examples of such disinfectants are chlorhexidine and quatemary ammonium compounds such as benzalkonium chloride.

According to an embodiment, the film-forming topical composition of the present invention further comprises other pharmacologically active ingredients whose presence is useful such as, for example healing agents.

Typical examples of healing agents used in the composition of the present invention are aloe vera, clostebol and hyaluronic acid.

In addition to water, the topical film-forming composition of the present invention may comprise other pharmaceutically acceptable vehicles provided they are miscible with water such as, for example, propylene glycol and glycerol.

Typically, the dry film obtained with the composition of the present invention has a thickness of from 0.1 to 5 mm, and even more typically it is of from 0.5 to 3 mm.

The composition of the present invention may further contain other conventional ingredients, such as , for example, preservatives, stabilizers, emulsifiers, buffers, pH adjusting agents, emollients, humectants and the like.

A typical example of a pH adjusting agent is lactic acid.

Examples of suitable forms for topical application of the composition of the present invention are sprays, fluid creams, ointments, gels, solutions and suspensions.

The following examples illustrate the present invention, without however limiting it in any way.

### EXAMPLE 1

| Preparation of a fluid cream | |
|---|---|
| Component | by weight % |
| A. polyvinyl alcohol | 10.00 |
| B. chitosan | 1.50 |
| C. propylene glycol | 15.00 |
| D. glycerol | 5.00 |
| E. benzalkonium chloride | 1.00 |
| F. aloe vera | 1.00 |
| G. lactic acid | 1.00 |
| H. purified water | qs. to 100 |

The aforesaid composition was prepared as follows:
a) dissolving, at 60°C while stirring continuously, of F in 40% of H;
b) dissolving, at 90°C, of A, B and G in 50% of H;
c) cooling the preceding phases a) and b), with continuous, gentle stirring,
d) adding E to the remaining 10% of H;
e) combining, while stirring, the solution obtained in step d) with that obtained in step a);
f) adding, while stirring, C and D to the solution obtained in b); and
g) combining, while stirring, the solutions of steps d) and e).

Tests performed by applying the aforesaid cream to the skin of volunteers showed that the mechanical properties of the thus obtained dry, transparent film remain unchanged for at least 24 hours.

When desired, the dry film is easily removed with water, and redness or other intolerance of the skin has not been encountered.

### TEST 1

### Elongation under load

Elongation under load was measured on dry films obtained from the following aqueous solutions:
A) polyvinyl alcohol at 10 by weight %;
B) chitosan at 1.5 by weight %; and
C) polyvinyl alcohol at 10 by weight % and chitosan at 1.5 by weight %.

The aforesaid solutions were placed on a glass plate of dimensions 7.5 x 2.5 cm, equal to an area of 18.75 cm².

The solutions were left to dry for 12 hours at room temperature (t = 25°C) and were then removed from the glass plate.

Measurement of the three films using a micrometer gave the following thickness values:
0.8 mm for the dry film obtained from solution A;
0.16 mm for the dry film obtained from solution B; and
2.1 mm for the dry film obtained from solution C.

The three dry films were then submitted to a gravimetric tension by means of three different loads: 0.5 g/cm², 1.0 g/cm² and 1.5 g/cm².

Elongation was measured at break.

The results are shown in Table 1.

**Table 1**

| Load (g/cm²) | elongation % film A | elongation % film B | elongation % film C |
|---|---|---|---|
| 0.5 | 23 | 5 | 25 |
| 1.0 | 39 | 7 | 36 |
| 1.5 | break | 8 | 44 |

### TEST 2

### Breaking load

The breaking load was measured on the dry films prepared according to the preceding Test 1.

The three dry films were then submitted to consecutive loads of 1 g/cm² for 30 seconds up to breaking of the film.

The results are shown in Table 2.

**Table 2**

| Film | Breaking load (g/cm²) |
|---|---|
| A | 1.5-2 |
| B | 7 |
| C | 7 |

The aforesaid Tables 1 and 2 show that the dry film obtained from aqueous solution A has sufficient thickness to guarantee a physical barrier, and good elastic properties within a limited range of tension. However, it proves to be fragile.

The dry film obtained from aqueous solution B has mechanical properties that are exactly opposite. Indeed, it has insufficient thickness to provide a cutaneous barrier, and low elasticity. On the other hand it exhibits greater resistance to the breaking load.

The dry film obtained from aqueous solution C of the invention has an optimum thickness, with elongation properties under load identical to film A for a stress equal to 1 g/cm² and better for a stress equal to 1.5 g/cm².

Moreover, its breaking load is equivalent to that of B.

## Claims

1. A topical film-forming aqueous composition for covering a skin lesion, **characterized in that** it comprises a mixture of a polyvinyl alcohol and a chitosan and is capable of forming a dry film *in situ.*

2. A composition according to Claim 1, wherein the amount of polyvinyl alcohol is of from 1 to 30 by weight %.

3. A composition according to Claim 2, wherein the amount of polyvinyl alcohol is of from 5 to 15 by weight %.

4. A composition according to any one of the claims from 1 to 3, wherein the amount of chitosan is of from 0.15 to 5 by weight %.

5. A composition according to Claim 4, wherein the amount of chitosan is of from 0.5 to 3 by weight %.

6. A composition according to any one of the claims from 1 to 5, wherein the said composition further comprises at least a disinfectant.

7. A composition according to Claim 6, wherein the said disinfectant is benzalkonium chloride or chlorhexidine.

8. A composition according to any one of the claims from 1 to 7, wherein the said composition further comprises a healing agent.

9. A composition according to Claim 8, wherein the said healing agent is selected from the group comprising aloe vera, clostebol and hyaluronic acid.

10. A composition according to any one of the claims from 1 to 9, in which the said dry film has a thickness of from 0.1 to 5 mm.
